# EUROPEAN PATENT APPLICATION

(11) **EP 3 604 169 A1**
(43) Date of publication of application: **05.02.2020**
(21) Application number: 18776330.5
(22) Date of filing: 07.03.2018
(51) Int. Cl.: B65D 81/20, A61J 1/10

(54) **STERILE PACKAGING CONTAINER AND MANUFACTURING METHOD THEREFOR**

(30) Priority: 30.03.2017 JP 2017068357
(71) Applicant: Nichiban Co., Ltd., Bunkyo-ku Tokyo 112-8663 (JP)
(72) Inventor: HIRAOKA, Takao, Tokyo 1128663 (JP); NAKAO, Kenichi, Tokyo 1128663 (JP)
(74) Representative: Sticht, Andreas
(86) International application number: PCT/JP2018/008851
(87) International publication number: WO 2018/180318

(57) **Abstract**

The present invention provides a sterile packaging container that can smoothly and repeatedly accommodate housed medicine without increasing the risk of contamination. The sterile packaging container comprises a front side film, and a back side film, wherein an opening is formed on one side, and two sides and a bottom are heat sealed, wherein the front side film is a non-breathable film with a strip of breathable sterile paper interposed and extending in the width direction, wherein the backside film is composed of a non-breathable film, wherein in the front side film, the vertical edges of the non-breathable film and the vertical edges of the breathable sterile paper have a structure in which they are heat-sealed to each other, and wherein all contact parts of non-breathable film and breathable sterile paper are heat sealed

## Description

### Technical Field

The present invention relates to a sterile packaging container to be subjected to gas sterilization such as ethylene oxide gas sterilization, and a method of manufacturing the same.

### Background Art

When aseptically packaging medicines etc., medicines are sealed in sterile packaging containers, and gas sterilization may be carried out with a sterilizing gas such as ethylene oxide gas, hydrogen peroxide gas, propylene oxide gas, formaldehyde gas, methyl bromide or the like. For sterile packaging containers to which such gas sterilization is applied, at least a portion of the sterile packaging container is gas permeable, so that a sterilization gas is able to penetrate the interior, and medicines or the like are stored inside; with the opening sealed, the contents are able to be sterilized by permeating the interior with a sterilizing gas from outside of the sterile packaging container.

### [Prior Art Document]

### [Patent document]

For example, as such sterile packaging containers which is sterilized by gas Patent Document 1 discloses a sterile container which is comprising a bag body having an non-breathable film in which an outer heat sealable layer, a support base layer, and an inner heat sealable layer are laminated in this order starting from the outside is heat-sealed at a predetermined place so that the inner heat sealable layer faces itself; and sterile paper made of breathable material that does not allow bacteria to pass is applied on the outer layer side of the bag body, wherein the side edges of the sterile paper are overlapped with the outer layer of the non-breathable film, are weakly sealed to form a cylindrical shape, and the peripheral part of the bag is lightly sealed or strongly sealed while leaving an opening part.

### [Prior Art Document]

### [Patent document]

[Patent Document 1] Japanese Patent Application Laid-open Publication No. 2006-347615

### Disclosure of Invention

### Problem to be Solved by the Invention

However, a single sheet, for example, may be loaded with multiple doses of medication, and in cases in which one dose of each drug is separated from the sheet and used, after removing a sheet loaded with a drug from a sterile packaging bag, the sterile packaging bag may be refilled with the sheet. However, with regard to the conventional sterile packaging container as described in Patent Document 1, FIG. 3 shows that at the junction between the sterile paper and the non-breathable film, because the edges of the non-breathable film or the sterile paper are free edges and protrude into the interior of the packaging container (the portions surrounded by the dotted lines), when a sheet is put back into the sterile packaging container, the edges of the sheet and the free edges of the non-breathable film come into contact with each other, and there is a risk that the sheets cannot smoothly be stored again, and the sheets and medicines may become damaged or contaminated.

Therefore, the present invention has been made in view of the above problems, and an object of the present invention is to provide a sterile packaging container capable of smoothly and repeatedly containing stored medicine without increasing the risk of contamination.

### Means to Solve the Problem

The inventors of the present invention conducted intensive studies in view of the above problems. As a result, in a sterile packaging container in which the edges of a front side film and a back side film are heat-sealed together, when providing a strip of breathable sterile paper extending in the width direction interposed between a non-breathable film constituting the front side film, according to the sterile packaging container in which all parts of the non-breathable film and breathable sterile paper in contact with each other are heat-sealed, the above problems are able to be solved, and the present invention has been accomplished. Specifically, the present invention provides the following.
(1) In an embodiment of the present invention, a sterile packaging container comprising a front side film, and a back side film, wherein an opening is formed on one side, and two sides and a bottom are heat sealed, wherein the front side film is a non-breathable film provided with a strip of breathable sterile paper interposed between and extending in the width direction, wherein the backside film is composed of a non-breathable film, wherein in the front side film, the vertical edges of the non-breathable film and the vertical edges of the breathable sterile paper have a structure in which they are heat-sealed to each other, and wherein all parts of the non-breathable film and breathable sterile paper in contact with each other are heat sealed.
(2) In a second embodiment of the present invention according to (1), wherein with respect to the front side film, the surface of the breathable sterile paper facing the back side film does not protrude beyond the surface of the non-breathable film facing the back side film.
(3) In a third embodiment of the present invention according to (1) or (2), wherein the non-breathable film constituting the front side film is an aluminum film laminated with a polyolefin film at least on the back side film facing surface, and the non-breathable film constituting the back side film is an aluminum film laminated with a polyolefin film on at least the front side film facing surface.
(4) In a fourth embodiment of the present invention according to (1) to (3), wherein a sealing means is provided at the opening.
(5) In a fifth embodiment of the present invention according to (1) to (4), wherein the breathable sterile paper is paper or a polyolefin non-woven fabric.
(6) In a sixth embodiment of the present invention according to (1) to (5), wherein the vertical dimension of the sterile packaging container is 10.0 cm or more and 18.0 cm or less, the vertical dimension of the breathable sterile paper is 2.0 cm or more and 6.0 cm or less, and the vertical dimension of the contact portion of non-breathable film and breathable sterile paper in the front side film is 0.3 cm or more and 1.5 cm or less.
(7) In a seventh embodiment of the present invention according to (1) to (6), wherein the contents of the sterile packaging container are patches that uses one sheet at a time, the patches are attached to and contained in a single peeling sheet, and after peeling a patch from the above-mentioned peeling sheet, the peeling sheet to which the unused patches are attached is housed in a sterile packaging container.
(8) In an eighth embodiment of the present invention, a manufacturing method of a sterile packaging container according to any one of embodiments 1 to 7 comprising arranging a heat-sealable surface of a non-breathable film of a front side film constituted by the non-breathable film and a strip-like breathable sterile paper which extends in the width direction in such a manner that the non-breathable film faces vertical edges of the breathable sterile paper and a heat-sealable surface of a back side film, and simultaneously heat-sealing two sides and a bottom of the front side film and the back side film, as well as the parts of the non-breathable film and the breathable sterile paper constituting the front side film.

### Advantages of the Invention

In the sterile packaging container of the present invention, when interposing a strip of breathable sterile paper extending in the width direction on the non-breathable film constituting the front side film, because the non-breathable film and all contacts of the breathable sterile paper are heat sealed, the end of the non-breathable film and the end of the breathable sterile paper do not protrude into the interior of the packaging container as free ends at the junction between the breathable sterile paper and the non-breathable film, and when a sheet is reaccommodated in the sterile packaging container, the end of the sheet and the free end of the breathable sterile paper or the non-breathable film can be smoothly reaccommodated without contact. For this reason, providing a sterile packaging container capable of smoothly and repeatedly containing stored medicines without increasing the risk of contamination is possible.

### Brief Description of Drawings

FIG. 1 is a general view of a sterile packaging container of the present invention.
FIG. 2 is a general view of a sterile packaging container of the present invention.
FIG. 3 is a view schematically showing a problem with inserting a sheet into a conventional sterile packaging container.
FIG. 4 is a view schematically showing how a sheet is inserted into the sterile packaging container of the present invention.
FIG. 5 of the present invention, (a) is a cross sectional view of when a breathable sterile paper and non-breathable sheet are not level, and (b) is a cross sectional view of when a breathable sterile paper and a non-breathable sheet are level.

### Mode for Carrying Out the Invention

Hereinafter, embodiments of the present invention will be described in detail with reference to the drawings.

### (Sterile Packaging Container)

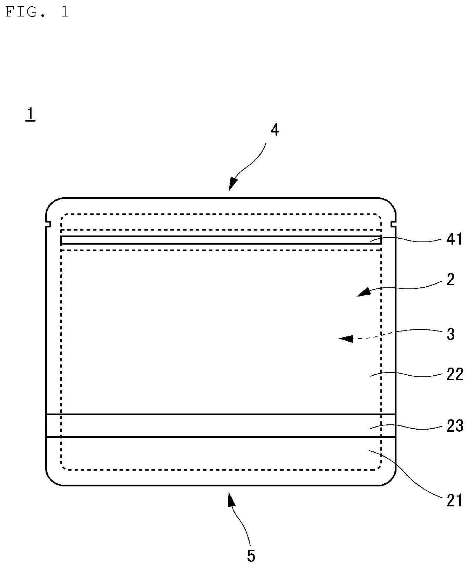

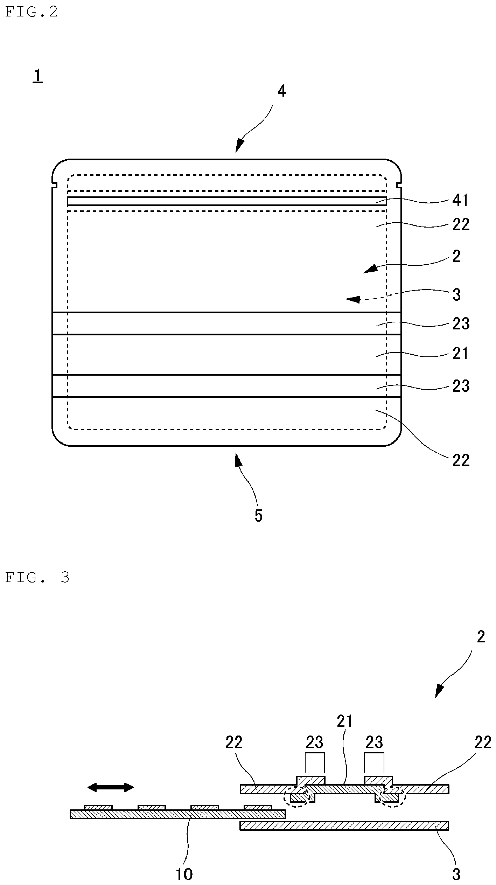

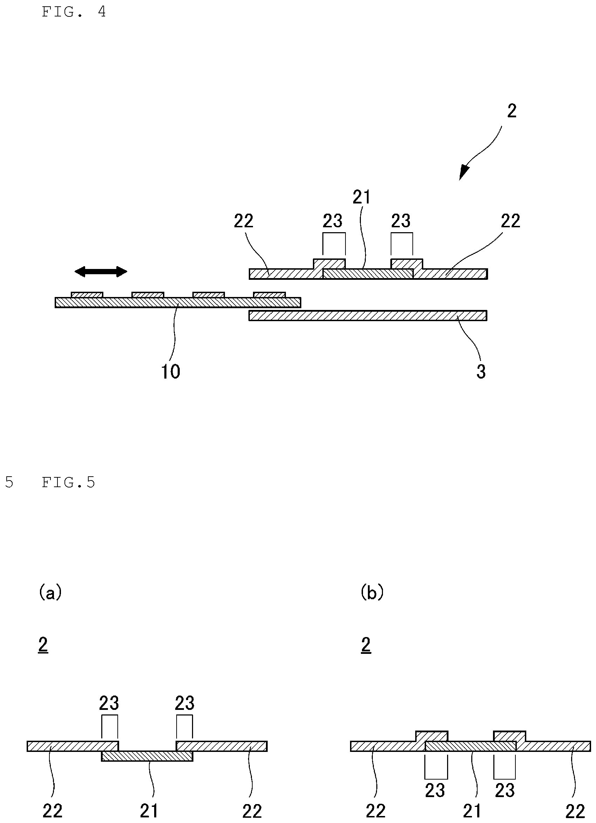

FIGs. 1 and 2 are general views of the sterile packaging container 1 of the present invention, and FIG. 4 is a schematic view showing how a sheet 10 is inserted into the sterile packaging container 1 of the present invention. The sterile packaging container 1 of the present invention is capable of forming an opening 4 at one side, and the bottom and two sides of a front side film 2 and a back side film 3 are bonded by heat sealing. The sterile packaging container 1 of the present invention is mainly for containing medicine, and is used to contain multiple dose loaded medicines in a single sheet 10. After removing a sheet 10 from the sterile packaging container 1 for one use, in order to save the medicine remaining on the sheet 10, such medicines are to be stored again in the sterile packaging container 1, and a sheet 10 is able to be removed from and re-accommodated in the sterile packaging container 1 a plurality of times. As described below, in the sterile packaging container 1 of the present invention, even when such medicine is re-accommodated, the edges of the sheet 10 do not get caught on the edges of the non-breathable film 22 or the edges of the breathable sterile paper 21, and because the sheet 10 is able to be smoothly stored, there is little risk of damaging the sheet 10 and/or the medicine, and the risk of contamination such as by bacteria can also be reduced as much as possible (Figure 4).

More specifically, the medicine, which are the contents contained in the sterile packaging container 1 of the present invention, are patches that use one sheet at a time, and examples of the patches can include medicines in which a plurality of (for example, 8 to 10) sheets which are attached to and stored in one release sheet. With respect to these types of patches, because avoiding contamination by bacteria etc. is necessary especially for patches applied to wounds, mucous membranes, and the skin in the vicinity of mucous membranes (e.g., ophthalmic transdermal patches), the patches are preferably housed in the sterile packaging container 1 of the present invention, sterilized by a gas sterilization process such as ethylene oxide gas sterilization, and aseptically provided; moreover, maintaining a clean state even after the sterile packaging container 1 has been opened is preferable. In the present invention, after peeling a patch from the peeling sheet removed from the sterile packaging container 1, by re-accommodating the peeling sheet to which the unused patches are attached in the sterile packaging container 1, the unused patches are substantially maintained in a clean state.

In the present invention, patches attached to such a peeling sheet are removed from the sterile packaging container 1 a plurality of times, and in order to re-accommodate the sterile packaging container 1 a plurality of times, the opening 4 is preferably provided with a sealing means 41. As such a sealing means 41, specifically a zipper can be mentioned, but any general-purpose means can be employed as the sealing means of a packaging container.

### [Front Side Film]

The front side film 2 is composed of a non-breathable film 22 and a breathable sterile paper 21 interposed therebetween as described later.

### (Non-Breathable Film Constituting the Front Side Film)

From these, the non-breathable film 22 prevents decomposition of the active ingredients such as the medicine contained inside of the sterile packaging container 1 and effectively blocks external impacts, and for the purpose of protecting the contents, the film material preferably has excellent light shielding properties, temperature shielding properties, and is durable, and more specifically, the film material preferably has a structure in which a plastic film is laminated to a metal foil.

Here, examples of the metal foil include metal foils made of aluminum, gold, copper, silver, tin, lead, zinc, titanium, stainless steel, nickel, beryllium, molybdenum, tantalum, iron, zirconium, and any alloys of these, but while any of these are possible, aluminum foil is preferably used due to stability and cost. In addition, polyethylene, polypropylene, polyethylene terephthalate, polyvinyl chloride, polycarbonate, polystyrene, polyacrylonitrile, ethylene-methacrylic acid copolymer, etc. can be used as the plastic film to be laminated on the aluminum foil, but from the viewpoint of durability, at least one of the outer layers is preferably a polyethylene terephthalate film rather than a metal foil. Also, in order for the non-breathable film 22 constituting the front side film 2 to be heat sealable with the breathable sterile paper 21 constituting the front side film 2 and the back side film 3, the outermost layer of the non-breathable film 22 constituting the front side film 2 is preferably made of a polyolefin film such as polyethylene or polypropylene. In particular, in the present invention, the non-breathable film 22 constituting the front side film 2 is preferably an aluminum film in which a polyolefin film is laminated on at least the surface facing the back side film 3.

The thickness of the non-breathable film 22 is preferably 30 µm or more and 200 µm or less, and more preferably 40 µm or more and 150 µm or less. By setting the thickness of the non-breathable film 22 in the above range, the durability of the sterile packaging container 1 is sufficiently maintained, the smooth loading and unloading (re-accommodation) of the contents into and out of the sterile packaging container 1 is secured, and also the openability of the sterile packaging container 1 is improved.

### (Breathable Sterile Paper)

In the present invention, a strip-like breathable sterile paper 21 extending in the width direction is interposed in the non-breathable film 22 constituting the front side film 2. By interposing the breathable sterile paper 21 between the non-breathable film 22 constituting the front side film 2, when gas sterilization is done with a sterilizing gas such as ethylene oxide gas, while the sterilizing gas around the sterile packaging container 1 passes through the breathable sterile paper 21, the contents of the sterile packaging container 1 can then be sterilized, and since the breathable sterile paper 21 itself is impervious to fungi, maintaining the sterility of the interior of the sterile packaging container 1 even after gas sterilization is possible. Furthermore, in the sterile packaging container 1 of the present invention, by using breathable sterile paper 21 for the front side film 2, the rigidity of the heat-sealed portion 23 of the breathable sterile paper 21 and the non-breathable film 22 can impart rigidity to the front side film 2, and at the same time the opening 4 is opened, the bottom 5 of the sterile packaging container 1 is able to be widened. Further, by incorporating the breathable sterile paper 21 in the front side film 2, inspection of the contents of the sterile packaging container 1 is facilitated.

Here, regarding the front side film 2, as shown in FIG. 2, the strip-like breathable sterile paper 21 extending in the width direction may be interposed in the vicinity of the vertical center portion of the front side film 2, or as shown in FIG. 1, a breathable sterile paper 21 may be present in the vicinity of the bottom 5 of the sterile packaging container 1. If the breathable sterile paper 21 is present in the vicinity of the vertical center portion of the front side film 2, with respect to the configuration of the front side film 2, a strip-like breathable sterile paper 21 is sandwiched between the non-breathable film 22 near the opening 4 and the non-breathable film 22 near the bottom 5, and if the breathable sterile paper 21 is present in the vicinity of the bottom 5 of the sterile packaging container 1, the breathable sterile paper 21 on the side of the bottom 5 is bonded to the non-breathable film 22 on the side of the opening 4. In all cases, the vertical edges of the non-breathable film 22 and the vertical edges of the breathable sterile paper 21 are heat-sealed to each other, and all of the parts of the non-breathable film 22 and the breathable sterile paper 21 in contact with each other are preferably heat-sealed.

In this way, by heat-sealing all the parts of the non-breathable film 22 and the breathable sterile paper 21 in contact with each other, neither the non-breathable film 22 nor the breathable sterile paper 21 form free edges at their contact surfaces, and free edges does not hinder smooth loading and unloading (reaccommodation) of the contents into the sterile packaging container 1.

Furthermore, in the present invention, as shown in FIG. 5(b), with respect to the front side film 2, the surface of the breathable sterile paper facing the back side film 3 preferably does not protrude beyond the surface of the non-breathable film 22 facing the back side film 3, and at the same time, the surface of the non-breathable film 22 facing the back side film 3 preferably does not protrude beyond the surface of the breathable sterile paper 21 facing the back side film 3. That is, in the present invention, in the front side film 2, the surface of breathable sterile paper 21 facing the back side film 3 and the surface of the non-breathable film 22 facing the back side film 3 are horizontally disposed on substantially the same plane; as a result, with respect to the contact surfaces of the breathable sterile paper 21 and the non-breathable film 22 in contact with each other of the front side film 2 facing the back side film 3 a protrusion as shown in FIG. 5(a) is not formed, and such a protrusion does not hinder smooth loading and unloading (reaccommodation) of the contents into the sterile packaging container 1.

As the breathable sterile paper 21, one made of conventionally known paper or nonwoven fabric can be used. Among them, the material of the non-woven fabric is not particularly limited, and those made of fibers such as nylon, polyester, polyacrylonitrile, polyolefin and polyurethane can be used, but a polyolefin non-woven fabric is preferably used in particular.

The thickness of the breathable sterile paper 21 is preferably 30 µm or more and 500 µm or less, and more preferably 80 µm or more and 300 µm or less. By making the thickness of the breathable sterile paper 21 in the above range, the ventilation gas of the sterilizing gas is sufficiently secured, smooth loading and unloading (re-accommodation) of the contents into the sterile packaging container 1 is secured, and the openability of the sterile packaging container 1 is also improved.

### [Back Side Film]

The back side film 3 is preferably made of a non-breathable film. Here, as the non-breathable film constituting the back side film 3, a film equivalent to the non-breathable film 22 constituting the front side film 2 can be used. That is, the non-breathable film which comprises the back side film 3 is preferably an aluminum film laminated with a polyolefin film at least on the surface facing the front side film 2.

### [Dimensions of Sterile Packaging Container]

The dimensions of the sterile packaging container 1 in the vertical direction is preferably 10.0 cm or more and 18.0 cm or less, and more preferably 13.5 cm or more and 16.5 cm or less, and on the lateral dimension is preferably 8.0 cm or more and 20.4 cm or less, and more preferably 10.0 cm or more and 18.7 cm or less. By making the dimensions of the sterile packaging container 1 within the above range, storing the contents becomes easy, and ease of openability is also improved. The dimension of the breathable sterile paper 21 in the vertical direction is preferably 2.0 cm or more and 6.0 cm or less, and more preferably 2.5 cm or more and 5.0 cm or less. By making the dimensions of the breathable sterile paper 21 in the above range, the permeability of the sterilizing gas at the time of gas sterilization is sufficiently secured, the costs due to using the breathable sterile paper 21 can also be minimized, and the ease of openability can be improved while maintaining the durability of the sterile packaging container 1. Further, the dimensions in the vertical direction of the contact portion (heat-sealed portion 23) between the breathable sterile paper 21 and the non-breathable film 22 is preferably 0.3 cm or more and 1.5 cm or less and is more preferably 0.5 cm or more and 1.2 cm or less. Generally, the larger the vertical dimension of the heat-sealed portion 23 is, the more the overall rigidity of the front side film 2 is improved, and the sterile packaging container 1 becomes easier to open.

### (Method for Manufacturing Sterile Packaging Container)

The sterile packaging container 1 of the present invention can be manufactured as follows. That is, the sterile packaging container 1 of the present invention is preferably manufactured by arranging the heat-sealable surface of the non-breathable film 22 of the front side film 2 constituted by the non-breathable film with the strip-like breathable sterile paper 21 which extends in the width direction in such a manner that the non-breathable film 22 faces the vertical edges of the breathable sterile paper 21 and the heat-sealable surface of the back side film 3, and simultaneously heat-sealing two sides and the bottom of the front side film 2 and the back side film 3, as well as the parts of the non-breathable film 22 with the breathable sterile paper 21 constituting the front side film.

In order to heat seal the areas of the breathable sterile paper 21 and the non-breathable film 22 in contact with each other simultaneously with heat sealing the front side film 2 and the back side film 3, with respect to the front side film 2, the surface of the breathable sterile paper 21 facing the back film 3 and the surface of the non-breathable film 22 facing the back side film 3 are horizontally positioned on the same plane. Note that in the above manufacturing method, the sterile packaging container 1 is manufactured in such a manner that the non-breathable film 22 constituting the front side film 2 is exposed to the outside with respect to the breathable sterile paper 21, but conversely, a method of manufacturing the sterile packaging container 1 in such a manner that the breathable sterile paper 21 is exposed to the outside with respect to the non-breathable film 22 is also within the scope of the present invention.

### Description of Reference Symbols

- 1: Sterile Packaging Container
- 2: Front Side Film
- 21: Breathable Sterile Paper
- 22: Non-Breathable Film
- 23: Heat-Sealed Portion
- 3: Back Side Film
- 4: Opening
- 41: Sealing Means
- 5: Bottom
- 10: Sheet

## Claims

1. A sterile packaging container comprising:
a front side film, and
a back side film,
wherein an opening is formed on one side, and two sides and a bottom are heat sealed,
wherein the front side film is a non-breathable film provided with a strip of breathable sterile paper interposed between and extending in the width direction,
wherein the backside film is composed of a non-breathable film,
wherein in the front side film, the vertical edges of the non-breathable film and the vertical edges of the breathable sterile paper have a structure in which they are heat-sealed to each other, and
wherein all parts of the non-breathable film and breathable sterile paper in contact with each other are heat sealed.

2. The sterile packaging container according to claim 1, wherein with respect to the front side film, the surface of the breathable sterile paper facing the back side film does not protrude beyond the surface of the non-breathable film facing the back side film.

3. The sterile packaging container according to claim 1 or 2, wherein the non-breathable film constituting the front side film is an aluminum film laminated with a polyolefin film at least on the back side film facing surface, and the non-breathable film constituting the back side film is an aluminum film laminated with a polyolefin film on at least the front side film facing surface.

4. The sterile packaging container according to any one of claims 1 to 3, wherein a sealing means is provided at the opening.

5. The sterile packaging container according to any one of claims 1 to 4, wherein the breathable sterile paper is paper or a polyolefin non-woven fabric.

6. The sterile packaging container according to any one of claims 1 to 5, wherein the vertical dimension of the sterile packaging container is 10.0 cm or more and 18.0 cm or less, the vertical dimension of the breathable sterile paper is 2.0 cm or more and 6.0 cm or less, and the vertical dimension of the contact portion of non-breathable film and breathable sterile paper in the front side film is 0.3 cm or more and 1.5 cm or less.

7. The sterile packaging container according to any one of claims 1 to 6, wherein the contents of the sterile packaging container are patches that uses one sheet at a time, the patches are attached to and contained in a single peeling sheet, and after peeling a patch from the above-mentioned peeling sheet, the peeling sheet to which the unused patches are attached is housed in a sterile packaging container.

8. A manufacturing method of the sterile packaging container according to any one of claims 1 to 7 comprising: arranging a heat-sealable surface of a non-breathable film of a front side film constituted by the non-breathable film and a strip-like breathable sterile paper which extends in the width direction in such a manner that the non-breathable film faces vertical edges of the breathable sterile paper and a heat-sealable surface of a back side film, and
simultaneously heat-sealing two sides and a bottom of the front side film and the back side film, as well as the parts of the non-breathable film and the breathable sterile paper constituting the front side film.
